# EUROPÄISCHE PATENTANMELDUNG

(11) **EP 2 260 764 A1**
(43) Veröffentlichungstag der Anmeldung: **15.12.2010**
(21) Anmeldenummer: 09162464.3
(22) Anmeldetag: 10.06.2009
(51) Int. Cl.: A61B 5/15, A61B 17/34, A61M 25/00, B21C 37/08, B21G 1/08

(54) **Mikronadel und Verfahren zu deren Herstellung**

(71) Anmelder: F.Hoffmann-La Roche AG, 4070 Basel (CH); Roche Diagnostics GmbH, 68305 Mannheim (DE)
(72) Erfinder: Haar, Hans-Peter, Dr., 69168 Wiesloch (DE)
(74) Vertreter: Pfiz, Thomas

(57) **Zusammenfassung**

Bei einem Verfahren zur Herstellung einer in Körpergewebe einstechbaren Mikronadel (12), bei welchem eine Nadelspitze (20) und ein vorzugsweise kapillarer Sammelkanal (14) für Köperflüssigkeit geformt wird, ist es vorgesehen, dass zumindest ein Teil eines aus einem Flachmaterial vorgefertigten Vorformlings (38) zu einer Rohrstruktur (22) umgeformt wird, so dass der Sammelkanal (14) im Bereich der Rohrstruktur (22) zumindest im Wesentlichen ringförmig geschlossen wird.

## Beschreibung

Die Erfindung betrifft ein Verfahren zur Herstellung einer in Körpergewebe einstechbaren Mikronadel, bei welchem eine Nadelspitze und ein vorzugsweise kapillarer Sammelkanal für Köperflüssigkeit geformt wird, wobei ein Vorformling aus einem Flachmaterial vorgefertigt wird. Die Erfindung betrifft weiter eine solchermaßen hergestellte Mikronadel.

Aus der WO 2008/122541 ist ein gattungsgemäßes Verfahren bekannt. Dort wird vorgeschlagen, einen kapillaraktiven Sammelbereich für Körperflüssigkeit durch zwei gegeneinander gefaltete Biegeteile zu definieren. Damit wird erreicht, dass der Benutzer nach einem Hauteinstich die Probennahme für eine Blutzuckermessung selbst nicht weiter kontrollieren muss. Zugleich wird eine herstellungstechnische Vereinfachung dahingehend erreicht, dass der Sammelbereich ohne aufwändige Materialbearbeitungsschritte geschaffen werden kann, wie sie etwa bei für den Materialabtrag bei einem Drahtmaterial erforderlich wären. Durch die Faltung wird allerdings eine nur halbseitig begrenzte U-förmige Kanalstruktur geschaffen, bei der die Verdunstung der Flüssigprobe während des Kapillartransports insbesondere bei mikroskopischen Entnahmemengen noch problematisch ist. Hierbei ist auch zu beachten, dass das Zeitfenster für die Entnahme und Messung nach unten begrenzt ist, um einen hinreichenden Messerfolg noch sicherzustellen.

Ausgehend hiervon liegt der Erfindung die Aufgabe zugrunde, die im Stand der Technik bekannten Verfahren und Vorrichtungen weiter zu verbessern und insbesondere für die Massenfertigung von analytischen Verbrauchsmitteln eine einfache Herstellbarkeit bei gleichzeitig hoher Gebrauchssicherheit zu gewährleisten.

Zur Lösung dieser Aufgabe wird die in den unabhängigen Patentansprüchen angegebene Merkmalskombination vorgeschlagen. Vorteilhafte Ausgestaltungen und Weiterbildungen der Erfindung ergeben sich aus den abhängigen Ansprüchen.

Die Erfindung geht von dem Gedanken aus, durch die Umformung von fertigungstechnisch vorteilhaften Flachteilen eine für den Einsatzzweck günstige 3D-Struktur zu schaffen. Dementsprechend wird erfindungsgemäß vorgeschlagen, dass zumindest ein Teil des Vorformlings zu einer Rohrstruktur umgeformt wird, so dass der Sammelkanal im Bereich der Rohrstruktur zumindest im Wesentlichen ringförmig geschlossen wird. Auf diese Weise wird ein besonders günstiges Verhältnis von Flüssigvolumen zu freier Oberfläche geschaffen, so dass die Verdunstung während der Probenverarbeitung deutlich reduziert werden kann. Durch die Rohrstruktur wird ggf. mit Ausnahme eines für die Verdunstung unbedeutenden Schließspalts eine umlaufende Begrenzung geschaffen, die auch für die Erhöhung der Kapillarität von Vorteil ist.

Vorteilhafterweise wird die Rohrstruktur unter Ausbildung eines längs durchlaufenden Schließspalts ringförmig gebogen, so dass ein zylindrischer Sammelkanal geschaffen wird. Hierfür ist es günstig, wenn zwei voneinander abgewandte Randkanten des Vorformlings auf Stoss zusammengebracht werden, wobei ggf. die Randkanten zumindest punktweise durch Verbindungsmittel, insbesondere durch Laserschweißstellen verbunden werden können.

Eine herstellungstechnische Vereinfachung ergibt sich dadurch, dass der Vorformling durch einen Formstempel in ein halboffenes Formnest eines Formwerkzeugs eingedrückt wird, wobei das Formnest eine Teilkontur der zu fertigenden Rohrstruktur definiert. Anschließend kann die Rohrstruktur durch einen auf freie Randkanten des Vorformlings aufgedrückten Schließstempel ausgeformt werden.

Alternativ ist es auch möglich, dass der Vorformling durch einen Ziehstein bzw. eine Matrize hindurchgezogen wird, wobei der Ziehstein eine die Kontur der Rohrstruktur definierende Öffnung aufweist.

Denkbar ist es auch, dass der Vorformling aus einer Kunststofffolie gebildet und durch Thermoformen in die Rohrstruktur umgewandelt wird.

Zur Kopplung mit einer automatischen Stech- und Messeinrichtung ist es vorteilhaft, wenn an dem Vorformling eine Haltestruktur, insbesondere proximal abstehenden Haltearme angeformt werden.

Eine weitere vorteilhafte Ausgestaltung sieht vor, dass eine Mehrzahl von Vorformlingen als zusammenhängender Verbund vorzugsweise durch Ätzen oder Laserschneiden vorstrukturiert werden.

Im Sinne einer erhöhten Systemintegration ist es weiterhin von Vorteil, wenn der Sammelkanal mit einem auf einen Inhaltsstoff der Körperflüssigkeit ansprechenden analytischen Testelement verbunden wird, so dass ein "One-Step-Handling" insbesondere für die Blutzuckerbestimmung ermöglicht wird.

Für eine verbesserte Anpassung an die Testgeometrie ist es günstig, wenn die Rohrstruktur mit einer von der Kreisform abweichenden, insbesondere elliptischen proximalen Öffnung versehen wird. In diesem Zusammenhang ist es auch denkbar, dass die Rohrstruktur insbesondere durch Faltlinien in dem Vorformling mit einer mehreckigen Kontur gebildet wird.

Um einen möglichst schmerzarmen Einstich zu ermöglichen, kann die Nadelspitze durch Ätzen, Schneiden oder Schleifen an dem Vorformling ausgebildet und/oder nach der Ausbildung der Rohrstruktur ausgearbeitet werden.

Für eine weiter verbesserte Flüssigkeitsaufnahme ist es vorteilhaft, wenn zumindest auf der Innenseite der Rohrstruktur eine hydrophile Schicht aufgebracht wird.

Gegenstand der Erfindung ist auch eine Mikronadel mit einer aus einem flachen Vorformling gebildeten und zumindest im Wesentlichen umlaufend geschlossenen Rohrstruktur.

Im Folgenden wird die Erfindung anhand der in der Zeichnung schematisch dargestellten Ausführungsbeispiele näher erläutert. Es zeigen
- Fig. 1: eine Mikronadel mit Sensoreinheit als dispo- sibler Microsampler zur Blutzuckerbestimmung in perspektivischer Ansicht;
- Fig. 2: die Mikronadel nach Fig. 1;
- Fig. 3: einen Abschnitt der Sensoreinheit nach Fig. 1 mit integrierten Lichtleitern;
- Fig. 4: die Sensoreinheit nach Fig. 1 mit stirnseitigem Testelement;
- Fig. 5: einen Verbund von Flachformteilen als Vorform- linge von Mikronadeln in der Draufsicht;
- Fig. 6 und 7: ein Formwerkzeug zur Umformung der Vor- formlinge zu Mikronadeln gemäß Fig. 2 in schau- bildlicher Darstellung.

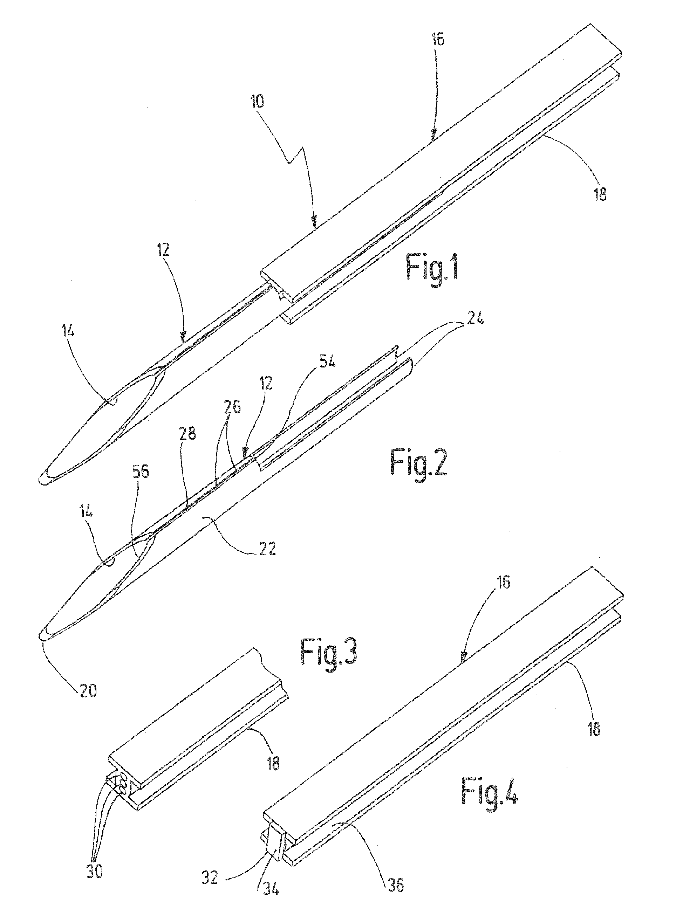

Die in Fig. 1 dargestellten Microsampler 10 lassen sich als Einmalartikel für eine Blutzuckermessung in einem dafür ausgebildeten Handgerät einsetzen, um eine mikroskopische Blutprobe zu gewinnen und vor Ort zu analysieren. Zu diesem Zweck umfassen die Microsampler 10 eine in die Haut bzw. Körpergewebe einstechbare Mikronadel 12, einen daran ausgebildeten Sammelkanal 14 zur Aufnahme von durch den Einstich erhaltener Körperflüssigkeit (Blut und/oder Gewebeflüssigkeit) und eine Sensoreinheit 16 für eine unmittelbare optische Probenvermessung. Die Sensoreinheit 16 weist einen mit einer Messeinrichtung koppelbaren Adapter 18 auf, wie es aus der WO 2008/122541 hervorgeht, auf die in diesem Zusammenhang Bezug genommen wird.

Wie am besten aus Fig. 2 ersichtlich, besitzt die Mikronadel 12 eine distale Nadelspitze 20 als Stechorgan, eine kapillare Rohrstruktur 22 zur umlaufenden Begrenzung des Sammelkanals 14 und zwei proximal abstehende Haltearme 24 zum Aufstecken auf den Adapter 18. Die Rohrstruktur 22 ist ringförmig gebogen, wobei die Randkanten 26 unter Begrenzung eines durchgehenden engen Schließspalts 28 auf Stoss zusammengeführt sind. Grundsätzlich ist es möglich, dass der Schließspalt 28 durch Laserschweißen zumindest punktweise überbrückt wird, so dass die Strukturstabilität noch weiter erhöht wird.

Fig. 3 zeigt den distalen Endabschnitt des Adapters 18 mit drei parallel verlaufenden integrierten Lichtleitern 30 für eine reflektometrische optische Messung. Zu diesem Zweck ist auf die distalen Lichtleiterenden ein Testelement 32 aufgebracht, wie es in Fig. 4 dargestellt ist. Zur Steckverbindung mit der Mikronadel 12 lassen sich deren Haltearme 24 in seitliche Aufnahmenuten 36 des Adapters 18 einführen. Im Messzustand steht das Testelement 32 über eine vorderseitige Nachweisschicht 34 in fluidischem Kontakt mit der proximalen Mündung des Sammelkanals 14, während über die Lichtleiter 30 eine rückseitige optische Erfassung einer Farbänderung möglich ist, die auf einer Reaktion mit Blutglukose beruht. Für eine sichere endständige Benetzung ist es wichtig, dass die Blutflüssigkeit ohne Verdunstungsverluste transportiert werden kann, was durch den im Wesentlichen seitlich geschlossenen Sammelkanal 14 gewährleistet ist.

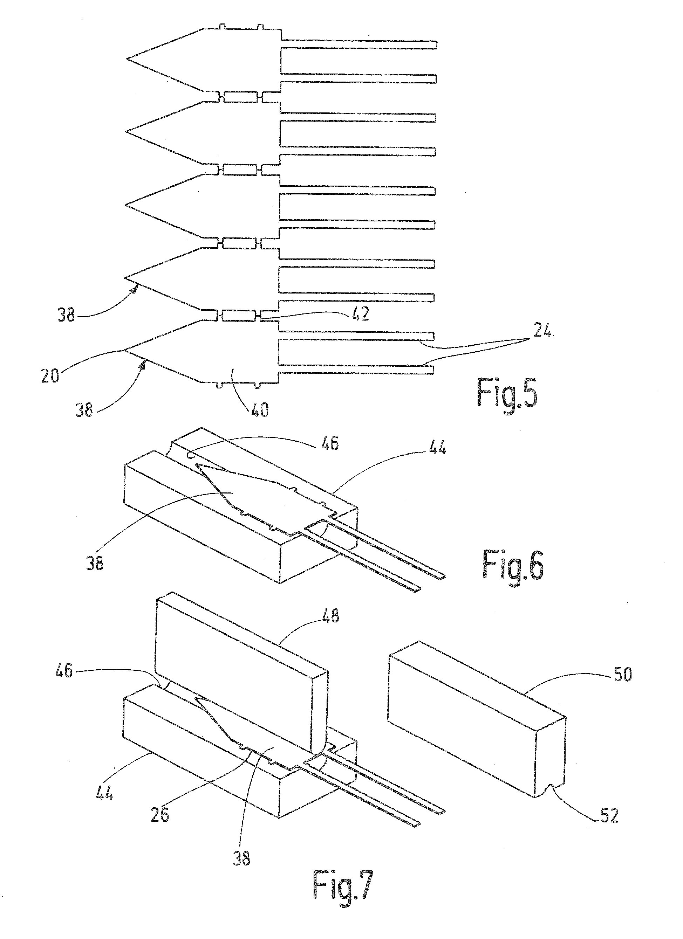

Um eine kostengünstige Massenfertigung zu ermöglichen, wird die Hohlstruktur aus einem Flachmaterial erzeugt, wie es in Fig. 5 bis 7 veranschaulicht ist.

Fig. 5 zeigt einen Verbund von Flachlanzetten als Vorformlinge 38, die beispielsweise aus einer Edelstahlfolie durch einen Ätzprozess oder Laserschneiden gebildet werden können, so dass die Nadelspitze 20, die Wandung 40 der Rohrstruktur 22 und die Haltearme 24 bereits in der Materialebene vorstrukturiert sind. Die Vorformlinge 38 können dabei noch über Materialbrücken 42 verbunden bleiben.

In einem nachfolgenden Umformprozess werden die Vorformlinge 38 vereinzelt auf einem Formwerkzeug 44 positioniert, wie es in Fig. 6 veranschaulicht ist. Das Formwerkzeug 44 weist ein halboffenes, etwa U-förmiges Formnest 46 auf, das eine Teilkontur der zu fertigenden Rohrstruktur definiert.

Die Biegeumformung erfolgt dann durch Stempel 48, 50 gemäß Fig. 7. Zunächst wird der Vorformling 38 durch den Formstempel 48 nach unten in das Formnest 46 eingedrückt, so dass die untere Halbseite der Rohrstruktur 22 geprägt wird. Sodann wird der Schließstempel 50 auf die nach oben überstehenden Randkanten 26 aufgedrückt, um die obere Halbseite der Rohrstruktur 22 entsprechend der Stirnkontur 52 des Schließstempels 50 ringförmig zu schließen. Das auf diese Weise erzeugte Formteil entspricht dann der Mikronadel gemäß Fig. 2. Gegebenenfalls kann die Nadelspitze 20 noch durch eine Nachbearbeitung, insbesondere durch einen Anschliff geschärft werden.

Die Rohrstruktur 22 kann einen durchgehenden kreisförmigen Querschnitt aufweisen. Zur Anpassung an die Rechteckform des Testelements 32 ist es auch möglich, den proximalen Bereich abzuflachen, so dass eine ovale bzw. elliptische Mündungsöffnung 54 erreicht wird. Durch die Wandverengung wird auch eine in Transportrichtung zunehmende Kapillarität erreicht, die eine sichere Benetzung des Testelements 32 begünstigt. In diesem Zusammenhang ist es auch günstig, wenn die Innenseite der Rohrstruktur 22 mit einer hydrophilen Schicht 56 versehen ist, die bereits im Zuge der Flachmaterialverarbeitung aufgebracht werden kann.

## Patentansprüche

1. Verfahren zur Herstellung einer in Körpergewebe einstechbaren Mikronadel (12), bei welchem eine Nadelspitze (20) und ein vorzugsweise kapillarer Sammelkanal (14) für Köperflüssigkeit geformt wird, wobei ein Vorformling (38) aus einem Flachmaterial vorgefertigt wird, **dadurch gekennzeichnet, dass** zumindest ein Teil des Vorformlings (38) zu einer Rohrstruktur (22) umgeformt wird, so dass der Sammelkanal (14) im Bereich der Rohrstruktur (22) zumindest im Wesentlichen ringförmig geschlossen wird.

2. Verfahren nach Anspruch 1, **dadurch gekennzeichnet, dass** die Rohrstruktur (22) unter Ausbildung eines längs durchlaufenden Schließspalts (28) ringförmig gebogen wird.

3. Verfahren nach Anspruch 1 oder 2, **dadurch gekennzeichnet, dass** zwei voneinander abgewandte Randkanten (26) des Vorformlings (38) auf Stoss zusammengebracht werden, und dass die Randkanten (26) zumindest punktweise durch Verbindungsmittel, insbesondere durch Laserschweißstellen verbunden werden.

4. Verfahren nach einem der Ansprüche 1 bis 3, **dadurch gekennzeichnet, dass** der Vorformling (38) durch einen Formstempel (48) in ein halboffenes Formnest (46) eines Formwerkzeugs (44) eingedrückt wird, wobei das Formnest (46) eine Teilkontur der zu fertigenden Rohrstruktur (22) definiert.

5. Verfahren nach einem der Ansprüche 1 bis 4, **dadurch gekennzeichnet, dass** die Rohrstruktur (22) durch einen auf freie Randkanten (26) des Vorformlings (38) aufgedrückten Schließstempel (50) ausgeformt wird.

6. Verfahren nach einem der Ansprüche 1 bis 3, **dadurch gekennzeichnet, dass** der Vorformling (38) durch einen Ziehstein hindurchgezogen wird, wobei der Ziehstein eine die Kontur der Rohrstruktur (22) definierende Öffnung aufweist.

7. Verfahren nach einem der Ansprüche 1 bis 3, **dadurch gekennzeichnet, dass** der Vorformling (38) aus einer Kunststofffolie gebildet und durch Thermoformen in die Rohrstruktur (22) umgewandelt wird.

8. Verfahren nach einem der Ansprüche 1 bis 7, **dadurch gekennzeichnet, dass** an dem Vorformling (38) eine Haltestruktur (24), insbesondere proximal abstehenden Haltearme angeformt werden.

9. Verfahren nach einem der Ansprüche 1 bis 8, **dadurch gekennzeichnet, dass** eine Mehrzahl von Vorformlingen (38) als zusammenhängender Verbund vorzugsweise durch Ätzen oder Laserschneiden vorstrukturiert werden.

10. Verfahren nach einem der Ansprüche 1 bis 9, **dadurch gekennzeichnet, dass** der Sammelkanal (14) mit einem auf einen Inhaltsstoff der Körperflüssigkeit ansprechenden analytischen Testelement (32) verbunden wird.

11. Verfahren nach einem der Ansprüche 1 bis 10, **dadurch gekennzeichnet, dass** die Rohrstruktur (22) mit einer von der Kreisform abweichenden, insbesondere elliptischen proximalen Öffnung (54) versehen wird.

12. Verfahren nach einem der Ansprüche 1 bis 11, **dadurch gekennzeichnet, dass** die Rohrstruktur (22) insbesondere durch Faltlinien in dem Vorformling (38) mit einer mehreckigen Kontur gebildet wird.

13. Verfahren nach einem der Ansprüche 1 bis 12, **dadurch gekennzeichnet, dass** die Nadelspitze (20) durch Ätzen, Schneiden oder Schleifen an dem Vorformling (38) ausgebildet und/oder nach der Ausbildung der Rohrstruktur (22) ausgearbeitet wird.

14. Verfahren nach einem der Ansprüche 1 bis 13, **dadurch gekennzeichnet, dass** zumindest auf der Innenseite der Rohrstruktur (22) eine hydrophile Schicht (56) aufgebracht wird.

15. Mikronadel hergestellt nach dem Verfahren gemäß einem der vorhergehenden Ansprüche, **gekennzeichnet durch** eine zumindest im Wesentlichen umlaufend geschlossene Rohrstruktur (22).
